# EUROPÄISCHE PATENTANMELDUNG

(11) **EP 3 954 995 A1**
(43) Veröffentlichungstag der Anmeldung: **16.02.2022**
(21) Anmeldenummer: 20190931.4
(22) Anmeldetag: 13.08.2020
(51) Int. Cl.: G01N 33/53, G01N 33/569

(54) **ERFINDUNG BETREFFEND MESSUNG DER CHILISCHÄRFE**

(71) Anmelder: Philipps-Universität Marburg, 35037 Marburg (DE)
(72) Erfinder: Rensing, Stefan, 79194 Gundelfingen (DE)
(74) Vertreter: Stumpf, Peter

(57) **Zusammenfassung**

Die Erfindung betrifft einen Antikörper gegen konjugiertes Capsaicin einen immunologischen ELISA-Test, um die Menge bzw. den Gehalt an Capsaicinoiden also die Chilischärfe in einer zu testenden Probe nachzuweisen. Damit ist es möglich in einer zu testenden Probe Capsaicinoide mit Colorimetrie, Chemilumineszenz oder Fluorometrie zuverlässig, einfach und kostengünstig mit bekannten Geräten und Vorrichtungen qualitativ und quantitativ zu bestimmen. In einer Ausführungsform ist der Test ein Lateral-Flowtest (LFT).

## Beschreibung

### Erfindung betreffend Messung der Chilischärfe

Die Erfindung betrifft das wissenschaftliche Gebiet der Analytik, insbesondere das Gebiet der Lebensmittelanalytik.

Die Deklarationspflicht von Lebensmitteln und die gestiegene Zahl der Allergien gegen bestimmte Inhaltstoffe in Lebensmitteln oder anderen Produkten macht es erforderlich, auf der Verpackung möglichst exakte Angaben über Art und Menge der Inhaltsstoffe zu machen. Dies ist z.B. bei der Angabe der Schärfe eines Lebensmittels sehr wichtig. Die Schärfe kommt z.B. von bestimmten Paprikasorten der Gattung Capsicum aus der Familie der Nachtschattengewächse (Solanaceae), die jedoch unterschiedliche Anteile an Capsaicinoiden aufweisen. Die Capsaicinoide Capsaicin und Dihydrocapsaicin sind die beiden Hauptscharfstoffe in Chilischoten. In geringerer Menge sind weiterhin vorhanden Nordihydrocapsaicin, Homocapsaicin und Homodihydrocapsaicin. Capsaicin (abgekürzt CPS) ist ein natürlich vorkommendes Alkaloid, das bei Säugetieren durch Wirkung auf spezifische Rezeptoren einen Hitze- oder Schärfereiz und damit verbunden die Freisetzung von Neuropeptiden wie Substanz P hervorruft. Capsaicin ist kein Aromastoff, da es keinen Geschmack hat, sondern es besitzt eine Reizwirkung auf die Schleimhäute. Die durchblutungssteigernde Wirkung wird z.B. in Wärmepflastern eingesetzt. Weiterhin wird die Reizwirkung von Capsaicin in Pfeffersprays zur Abschreckung von Tieren und Menschen verwendet.

### Stand der Technik

Bislang wird die Schärfe durch das sogenannte Schärfeempfinden einer Person definiert und es existieren viele verschiedene Skalen, um die Schärfe von Chilis anzugeben. Eine recht gebräuchliche Skala ist die von Wilbur L. Scoville entwickelte Scoville-Skala, eine subjektiv bestimmte Einordnung. Dabei wird versucht, sehr scharfe Lebensmittel durch stetiges Verdünnen besser einstufen zu können. Der Grad der Verdünnung, bei dem keine Schärfe mehr festzustellen ist, wird als Scoville-Grad, SCU für Scoville Units, auch: SHU für Scoville Heat Units angegeben. Paprika ohne feststellbare Schärfe haben den Scoville-Grad 0, reines Capsaicin entspricht 16.000.000 Scoville. Die Capsaicinoide Capsaicin und Dihydrocapsaicin sind nach der Scoville-Skala fast doppelt so scharf wie die in geringerer Menge vorhandenen Capsaicinoide Nordihydrocapsaicin, Homocapsaicin und Homodihydrocapsaicin. Die Ermittlung der Schärfe mittels Scoville-Skala hat jedoch einige bedeutende Einschränkungen: Zum einen besitzt jeder Mensch eine unterschiedliche Toleranz gegenüber Capsaicinoiden, zum anderen wird durch ständige Capsaicinoidaufnahme diese Toleranzschwelle heraufgesetzt, es findet ein Adaptionsprozess statt. Somit ist für das Ergebnis nicht nur die Auswahl der Probanden ausschlaggebend, sondern auch, wie viele Einzeltests bereits mit einem einzelnen Probanden durchgeführt wurden.

Unter anderem in Restaurants und auf den Verpackungen einiger Lebensmittel findet man noch verschiedene andere, meist frei erfundene Schärfeskalen. So wird zunehmende Schärfe durch eine höhere Anzahl an abgebildeten Chilis dargestellt oder durch unterschiedliche Farbgebung unterstützt. Beispielsweise kann ein grüner Chili als mild gelten, zwei gelbe als mäßig scharf und drei rote Chilis als scharf. Das heute in der modernen Lebensmittelanalytik gebräuchliche Verfahren zur Bestimmung der Chilischärfe ist die Hochleistungsflüssigkeits-Chromatographie (HPLC). Dieses Verfahren identifiziert und misst z. B. die Konzentration der verschiedenen Hitze bzw. Schärfe erzeugenden Capsaicinoide. Dabei werden die prozentualen Anteile der zwei häufigsten Capsaicinverbindungen (Capsaicin, Dihydrocapsaicin) sowie gelegentlich noch Nordihydrocapsaicin gemessen. Die Messungen der einzelnen Stoffe werden dabei bezüglich ihrer relativen Schärfe bzw. Hitzeerzeugung gewichtet. Aufgrund des Bekanntheitsgrades der Scoville-Skala werden die Ergebnisse der HPLC, die eigentlich in Schärfeeinheiten der American Spice Trade Association (ASTA) angegeben werden, in der Regel in das Scoville-Bezugssystem umgerechnet. Die Umrechnung ist jedoch nur annähernd genau und liefert meist zu geringe Scoville-Werte.

Die Nachteile des HPLC-Verfahrens sind, dass es sehr teuer und aufwendig ist und nur von Fachpersonal durchgeführt werden kann. Für eine Routineanalytik eignet es sich nicht.

Andere typische Verfahren wie massenspektrometrische Verfahren, chromatographische Verfahren wie z.B. Gaschromatographie oder flüssigkeitsbasierende Verfahren mit Festphasentrennung oder mikro- und nanofluidische Verfahren haben den Nachteil, dass sie aufwendige Apparaturen benötigen, teuer und ebenfalls nicht für Routineanalytik geeignet sind.

Eine typische Nachweismethode in der Lebensmittelanalytik ist der ELISA-Test (Enzyme-linked Immunosorbent Assay) von dem es mittlerweile viele spezifische Ausführungsformen als Radioimmunassay (RIA), Elektrochemilumineszenz (ECL)-Immunassay, CLIA (chemoluminescence-linked immunosorbent assay), FLIA (fluorescence-linked immunosorbent assay) oder Multiplex-Assay gibt.

Mit Hilfe eines Immunassays kann die Menge eines Stoffes qualitativ und quantitativ bestimmt werden. Er bietet neben hoher Sensitivität und Spezifität, den Vorteil einer möglichen Automatisierung und ist somit für den Routinealltag besonders gut geeignet. Voraussetzung ist allerdings das Vorhandensein eines geeigneten Antikörpers für den nachzuweisenden Stoff.

### Aufgabe

Aufgabe der vorliegenden Erfindung ist es daher, die Nachteile im Stand der Technik zu überwinden und ein verbessertes, zuverlässiges und einfach durchzuführendes Verfahren zur Bestimmung der Chilischärfe bereitzustellen.

### Lösung der Aufgabe

Diese Aufgabe wird erfindungsgemäß durch ein Verfahren gemäß Anspruch 1 gelöst, weitere vorteilhafte Ausführungsformen und Weiterbildungen der Erfindung ergeben sich aus den Unteransprüchen.

Erfindungsgemäß wird ein Verfahren zur Bestimmung der Chilischärfe einer Probe mit Hilfe eines ELISA-Tests bereitgestellt. Die Erstellung eines ELISA war insbesondere durch das Fehlen von geeigneten Antikörpern nicht möglich. Die Herstellung von Capsaicin-Antikörper wird dadurch erschwert, dass Capsaicin ein sehr kleines Molekül ist. Um immunogen zu sein, muss das Capsaicin daher an ein anderes Molekül (Protein) gekoppelt werden, bevor es dem Immunsystem präsentiert wird. Die Wahl des geeigneten Moleküls zur Kopplung (Konjugation), sowie die Art der Kopplung ist hierbei wichtig. Auch musste ein geeigneter Organismus zur Bildung der Antikörper gefunden werden, denn Säuger reagieren mittels einer neurologischen Antwort auf die Gabe von Capsaicin. Die intravenöse Gabe zur Immunisierung an den Organismus muss ideal sein und ein Zuviel kann je nach Menge zum Tode führen. Die Art der Gabe des immunogenen Agens und die Wahl der Organismus sind daher wesentlich und relevant.

Überraschenderweise ist es gelungen geeignete Antikörper gegen Capsaicinoide insbesondere gegen Capsaicin, Dihydrocapsaicin, Nordihydrocapsaicin, Homocapsaicin und Homodihydrocapsaicin bereitzustellen und einen immunologischen ELISA-Test zu entwickeln, um die Menge bzw. den Gehalt an Capsaicinoiden, also die Chilischärfe, in einer Probe nachzuweisen. Damit ist es jetzt möglich, Capsaicinoide colorimetrisch, fluorimetrisch und mit Chemilumineszenz mit bekannten Geräten und Vorrichtungen zu bestimmen. Diese Art der Bestimmung der Chilischärfe erfolgt besser, günstiger und schneller als mit HPLC und ist wesentlich zuverlässiger, als die subjektiv ermittelte Scoville-Einteilung.

Der erfindungsgemäße ELISA-Test bietet, neben hoher Sensitivität und Spezifität, die Möglichkeit der Automatisierung und ist somit für den Einsatz in der Lebensmittelanalytik besonders gut geeignet.

Das Verfahren zur Bestimmung der Chilischärfe einer Probe mittels eines ELISA-Tests umfasst folgende Schritte:
- Bereitstellen einer Mikrotiterplatte, die in mindestens einer Vertiefung mit konjugiertem Capsaicin beschichtet ist
- Blockierung der unspezifischen Bindungsstellen durch Zugabe von Blocklösung in die mindestens eine Vertiefung
- Zugabe der zu testenden Probe in die mindestens eine Vertiefung der Mikrotiterplatte
- Zugabe eines ersten Antikörpers in die mindestens eine Vertiefung, so dass der erste Antikörper konjugiertes Capsaicin in der Beschichtung und freie Capsaicinoide aus der zu testenden Probe bindet
- Erstes Waschen der mindestens einen Vertiefung, so dass nicht gebundener erster Antikörper entfernt wird
- Zugabe eines zweiten enzymgekoppelten Antikörpers in die mindestens eine Vertiefung der Mikrotiterplatte, so dass der zweite Antikörper an den ersten Antikörper bindet
- Zweites Waschen der mindestens einen Vertiefung der Mikrotiterplatte, so dass nicht gebundener zweiter Antikörper entfernt wird
- Zugabe von Substrat in die mindestens eine Vertiefung der Mikrotiterplatte, sodass es durch das am zweiten Antikörper gekoppelte Enzym zu einer enzymatischen Reaktion in ein Produkt kommt
- Detektion und Quantifizierung des Produktes und Ermittlung der Chilischärfe aus der zu testenden Probe

Anhand eines Kontrollansatzes ohne Capsaicinoide in der zu testenden Probe wird die maximale Menge an Produkt ermittelt. Die Konzentration an Capsaicinoiden in der zu testenden Probe erfolgt dann anhand der Abnahme des Produktes im Vergleich zum Kontrollansatz. Ist die Menge an Produkt geringer als im Kontrollansatz waren in der zu testenden Probe Capsaicinoide enthalten. Je weniger Produkt messbar ist, desto höher war die Konzentration der Capsaicinoide in der zu testenden Probe. Zusätzlich wird mittels einer Standardreihe mit definierter Capsaicin Konzentration die Konzentration an Capsaicinoiden und damit der Chilischärfe in der zu testenden Probe anhand der Abnahme des Produktes ermittelt.

In einer Ausführungsform der Erfindung ist der zweite Antikörper mit Biotin markiert und es erfolgt eine separate Zugabe eines an Streptavidin gekoppelten Enzyms. Alternativ und bevorzugt ist der zweite spezifische Antikörper direkt mit Enzym (z.B. Meerrettichperoxidase) gekoppelt. Zur Verstärkung des Signals wird in einer besonderen Ausführungsform ein gegen den zweiten spezifischen Antikörper gerichteter dritter Antikörper, der mit Enzym gekoppelt ist, verwendet. Das Enzym ist vorzugsweise eine Peroxidase oder alkalische Phosphatase. Zur Detektion wird das jeweilige Enzym mit einem geeigneten Substrat umgesetzt, das mit Colorimetrie, Fluorimetrie oder Chemilumineszenz nachweisbar ist. Diese Vorgehensweise kennt der Fachmann.

Da es problematisch ist, Antikörper gegen Capsaicin in Säugetieren herzustellen, weil diese auf Capsaicinoide reagieren und einen spezifischen Rezeptor aufweisen, handelt es sich beim erfindungsgemäßen Antikörper gegen Capsaicinoide um polyklonale Antikörper insbesondere um affinitätsgereinigtes IgY Antiserum, das in Hühnern (Chicken) produziert wurde, da Vögel nicht auf Capsaicin reagieren und keinen spezifischen Capsaicin-Rezeptor aufweisen.

Bei der zu testenden Probe handelt es sich z.B. um Pflanzenextrakt, Paprika- oder Chilischoten der Gattung Capsicum. Die Probe ist auch ein Lebensmittel, das Pflanzenextrakt der Gattung Capsicum umfasst oder umfassen könnte. Die Probe ist auch z.B. eine Wärmepflaster oder Pfefferspray, die evtl. Pflanzenextrakt der Gattung Capsicum umfassen. Hier ist es wichtig, den Schärfegrad also die Chilischärfe genau zu bestimmen, da Wärmepflaster oder Pfefferspray in direkten Kontakt mit Menschen oder Säugetieren kommen. Die Probe ist fest oder flüssig, wobei eine feste Probe zum Einsatz in der Analytik verflüssigt wird. Dies erfolgt nach gängigen Methoden, die der Fachmann kennt.

### Ausführungsbeispiele

Als Materialien werden beispielhaft verwendet:
Erster Antikörper: Antikörper gegen konjugiertes Capsaicin
Zweiter Antikörper: Rabbit anti-Chicken IgY

Alternativ wird als Zweiter Antikörper Goat anti-Chicken IgY eingesetzt, um die unspezifische Bindung zu reduzieren und damit die Sensitivität zu erhöhen
Capsaicin konjugiert mit BSA
Capsaicin
BSA
Mikrotiterplatte

Als Puffer wird eingesetzt:
10x PBS-Tween 20 [0,1M PBS, 0,05% Tween 20 (pH7,4)] erhitzen

| | |
|---|---|
| Na₂HPO₄ (wasserfrei) | 10,9g |
| NaH₂PO₄ (wassserfrei) | 3,2g |
| NaCl | 90,0g |
| Destilliertes Wasser | ad 1000ml |

Die Einzelbestandteile werden in vorgelegtem Wasser gelöst, der pH-Wert auf 7.4 eingestellt und auf 1000 ml aufgefüllt. Anschließend wird 0,5ml Tween 20 zugegeben und die Lösung bei 20-25 Grad Celsius gelagert.

Die ELISA Blocklösung umfasst 2% fettfreies Milchpulver gelöst in PBS (phosphatgepufferte Salzlösung, Englisch: phosphate buffered saline). Zusätzlich wird 0,05% Tween20 zugegeben, sodass gegeben ist: 2g Milchpulver/100ml PBS 0,05%Tween 20.

Der ELISA Waschpuffer umfasst 1% NaCl (10g/L) und 0,05% Tween 20 (2,5ml/5L) (0,5ml/L).

Der ELISA Beschichtungspuffer umfasst 0,1M NaHCO3 (8,4g/L).

Die Angaben sind beispielhaft zu verstehen, es ist dem Fachmann bekannt, wie die Mengenangaben bei größerem oder kleinerem Volumen anzupassen sind.

### Bereitstellen einer Mikrotiterplatte, die in mindestens einer Vertiefung mit konjugiertem Capsaicin beschichtet ist

Zur Beschichtung wird eine handelsübliche Mikrotiterplatte mit Vertiefungen (Wells) verwendet, z.B. eine 96 Well Platte. In einem Kontrollansatz wird BSA (Bovines Serumalbumin) mit ELISA Beschichtungspuffer auf eine Konzentration von 0,02 mg/ml verdünnt. In einem anderen Ansatz wird BSA-konjugiertes Capsaicin mit ELISA Beschichtungspuffer auf eine Konzentration von 0,02 mg/ml verdünnt. Ein geeignetes Volumen z.B. 120 µL wird pro Vertiefung (Well) aufgetragen und die Mikrotiterplatte beschichtet. Die Mikrotiterplatte wird abgedichtet, sodass keine Flüssigkeit evaporieren kann z.B. mit einer Folie oder einem Deckel. Anschließend wird die Mikrotiterplatte mindestens 2 Stunden bei ca. 37°C inkubiert, bis die Beschichtung vollständig abgeschlossen ist.

Die so mit Capsaicin beschichtete Mikrotiterplatte kann sofort verwendet oder bei 2-8°C gelagert und dann verwendet werden. Es wird eine Lagerbeständigkeit von maximal 7 Tagen bei gleichbleibender Qualität ermittelt.

### Blocken der unspezifischen Bindestellen

Zum Verwenden der Platte wird der Beschichtungspuffer aus den Wells der Mikrotiterplatte entfernt. Anschließend werden 200 µL ELISA Blocklösung pro Vertiefung der Mikrotiterplatte (Well) aufgetragen. Die Blocklösung bewirkt, dass eine unspezifische Bindung an die Bindestellen am Capsaicin verhindert wird. Die Mikrotiterplatte wird anschließend erneut abgedichtet, sodass keine Flüssigkeit evaporieren kann und bei 2-8°C mindestens 4 Stunden inkubiert.

### Zugabe der zu testenden Probe

### Vorbereitung der Probe

Dazu wird die zu Probe, z.B. 0,5 g getrocknete Chili zerkleinert, z.B. gemahlen und verflüssigt, z.B. in ein 15 ml Zentrifugenröhrchen mit 5 ml demineralisiertes, deionisiertes, vollentsalztes Wasser (=VE Wasser) transferiert und mindestens 2 Stunden bei 20-25°C inkubiert. Danach wird die zu messende Probe so zentrifugiert, dass ein Überstand und ein Pellet entstehen, dies erfolgt z.B. für ca. 15 min bei 5000 rpm. Der Überstand wird in ein neues geeignetes Gefäß transferiert und für den ELISA verwendet. Flüssige oder aufgeschlämmte Lebensmittelproben werden ebenfalls zentrifugiert und der Überstand verwendet. Andere feste Proben werden zunächst zerkleinert und mit geeigneten Methoden verflüssigt, sodass sie zentrifugiert werden können.

Die Applikation der zu testenden Probe erfolgt indem zunächst 80 µL PBS Tween 20 pro Well aufgetragen werden. Anschließend
- 20 µL des Überstand der zu messenden Probe pro Well aufgetragen werden
- 20 µL VE Wasser pro Well pipettiert werden, die kein Capsaicinoid enthält (Positivkontrolle, d.h. maximales Signal des beschichteten konjugierten Capsaicins wird erzielt)
- 10 µL einer 1:110 Verdünnung des Anti-Capsaicin Antikörpers als Erstantikörper in PBS-Tween 20 pro Well zugegeben werden.

### Erstes Waschen der Mikrotiterplatte

Dazu wird die ELISA Blocklösung entfernt und die Vertiefungen der Mikrotiterplatte (Wells) werden mit einer geeigneten Menge an Waschpuffer gewaschen, z.B. mit 200 µL Waschpuffer. Dazu wird die mit Waschpuffer befüllte Mikrotiterplatte geschüttelt, z. B. für 7 min auf einen Taumelschüttler gestellt. Dieser Waschschritt kann wiederholt werden bevorzugt ist eine Wiederholung von dreimal. Zur Inkubation wird die Mikrotiterplatte z.B. für 70 Minuten auf einem Taumelschüttler bei 20-25°C geschüttelt, damit sich die Flüssigkeiten im Well gleichmäßig verteilen und alle Bindestellen gut von Flüssigkeit benetzt sind. Anschließend werden zusätzlich 110 µL der Flüssigkeit abgenommen und die Wells werden mit 120 µL ELISA Waschpuffer erneut gewaschen. Dazu wird die mit Waschpuffer gefüllte Platte für 7 min auf einen Taumelschüttler gestellt. Dieser Waschschritt kann wiederholt werden z.B. dreimal.

### Zugabe eines zweiten enzymgekoppelten Antikörpers in die Vertiefung der Mikrotiterplatte

Dazu wird 110 µl des 1:500 verdünnten Zweitantikörpers z.B. ein Rabbit anti-Chicken IgY in PBS Tween 20 pro Well aufgetragen. Als Kontrolle werden 110 µL PBS Tween 20 in die Wells gegeben, die keinen Zweitantikörper erhalten sollen. Die Mikrotiterplatte wird inkubiert bis die Reaktion des Zweitantikörpers abgeschlossen ist, z.B. 30 Minuten auf einem Taumelschüttler bei 20-25 °C.

### Zweites Waschen der Mikrotiterplatte

Dazu werden 110 µL Flüssigkeit abgenommen und die Wells mit 120 µL ELISA Waschpuffer gewaschen. Beispielhaft erfolgt dies für 7 Minuten auf einem Taumelschüttler. Dieser Waschschritt kann wiederholt werden z.B. dreimal.

### Zugabe von Substrat, sodass es durch das am zweiten Antikörper gebundene Enzym zu einer enzymatischen Reaktion in ein Produkt kommt

Die Substrat Zugabe erfolgt, indem z.B. TMB- (3,3',5,5'-Tetramethylbenzidin) Substrat pro Well zugegeben wird, z.B. 110 µl TMB, sodass eine Farbreaktion erfolgt. Die Mikrotiterplatte wird dazu z.B. für 15-45 Minuten bei 20-25°C inkubiert, bis sich der gewünschte Farbumschlag entwickelt hat.

### Detektion und Quantifizierung des Produktes und Ermittlung der Chilischärfe aus der zu testenden Probe

Die Umsetzung des Substrates wird z.B. durch Zugabe von Schwefelsäure gestoppt, z.B. durch 110 µL einer 2M Schwefelsäure. Die Absorption der Farbe wird bei einer Wellenlänge von 450 nm mit Hilfe eines Plate Readers detektiert und quantifiziert. Durch Mitlaufen einer Standardreihe kann die Capsaicinmenge in der zu testenden Probe ermittelt werden. Dazu wird eine Probenreihe unterschiedlicher definierter Capsaicinkonzentrationen im ELISA mitgeführt, z.B. verschiedene Konzentrationen von 0,01 mg/ml bis 0,1 mg/ml.

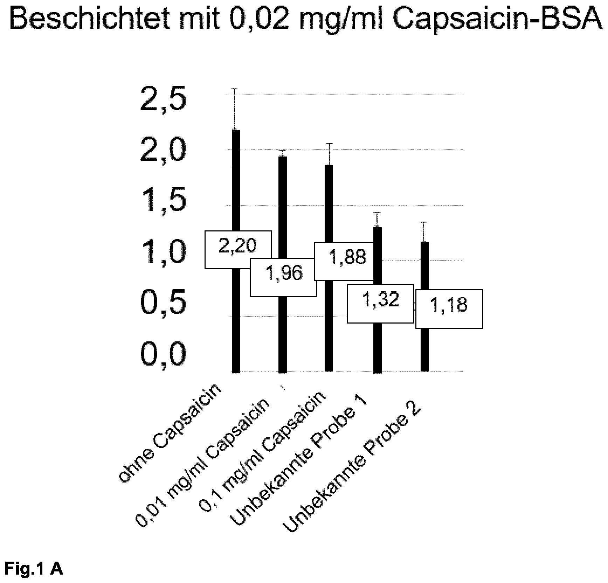

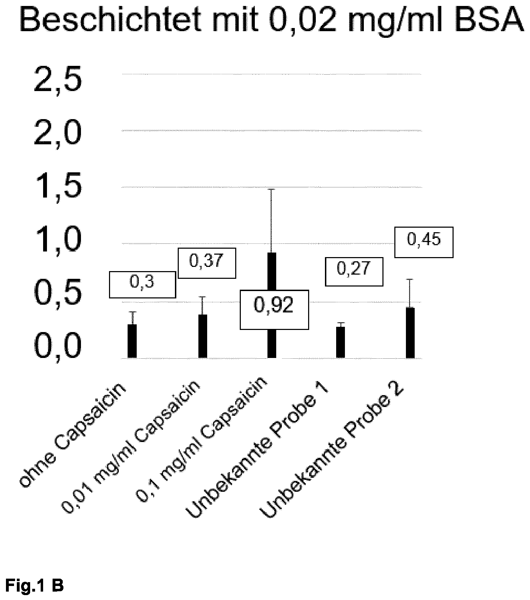

**Fig. 1** **A** zeigt Ergebnisse des oben beschriebenen kompetitiven ELISAs, zur Bestimmung von Chilischäfe anhand der Capsaicinoid-Mengen in zwei unbekannten Proben (1 und 2). Von allen dargestellten Werten wurde ein Leerwert abgezogen, der durch das Messen von Wells entstanden ist, denen weder Capsaicinoid noch Antikörper zugegeben wurde. Hier wurden alle Proben in Triplikaten gemessen. Ohne Capsaicinpid wird das größte Signal erhalten. Sobald Capsaicin in verschiedenen Konzentrationen hier beispielhaft 0,01 mg/ml und 0,1 mg/ml zugegeben wird, konkurriert das zugegebene Capsaicinoid mit dem gebundenen Capsaicin am Well um den primären Antikörper. In de Folge wird weniger des primären Antikörpers am Well gebunden und das Signal wird schwächer. Je größer die zugegebene Capsaicin bzw. Capsaicinoid-Menge ist, desto kleiner wird das Signal. Dies ist beispielhaft für 0,01 mg/ml und 0,1 mg/ml eines definierten Capsaicinstandards zu sehen, danach folgen zwei Proben (1 und 2) mit unbekannter zu messender Capsaicinmenge (Chiliextrakt).

**Fig. 1** **B** zeigt die Ergebnisse der Kontrolle. Hier wurden die Wells nicht mit BSA-konjugiertem Capsaicin, sondern lediglich mit BSA beschichtet, wodurch unspezifische Signale detektiert werden.

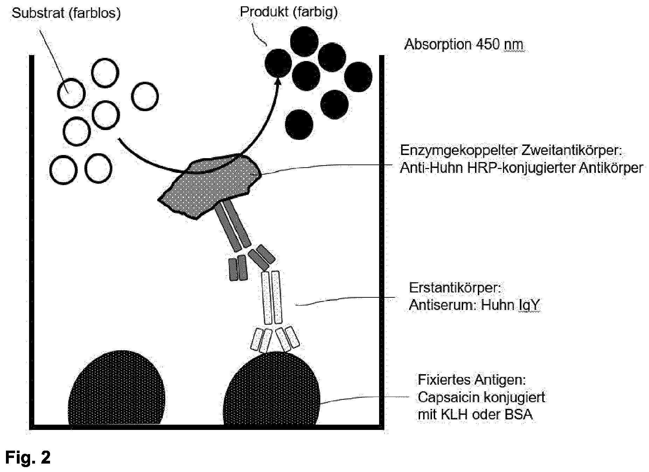

**Fig. 2** zeigt das Prinzip des ELISA zum Nachweis von Chilischärfe. Mit BSA oder KLH (=keyhole limpet hemocyanin) konjugiertes Capsaicin wird als Standardantigen mit bekannter Menge auf eine Oberfläche aufgebracht. An dieses Capsaicin bindet das Antiserum aus Huhn, welches seinerseits durch einen Anti-Huhn Antikörper erkannt wird, an den ein Enzym (HRP Meerrettichperoxidase) gekoppelt ist. Das Enzym setzt das Substrat um, es entsteht ein farbiges Produkt, dessen Farbintensität optisch gemessen wird, z.B. durch Absorption bei 450 nm. Im kompetitiven Ansatz wird zusätzlich zum Standardantigen die unbekannte Probe dazugeben. sind Capsaicinoide enthalten, konkurrieren diese mit dem festgebundenen um die Bindungsstellen des ersten Antikörpers, so dass ein verringertes Signal gemessen wird. Die Menge an Capsaicinoiden aus der zu testenden Probe wird durch Absorptionsmessung ermittelt. Es ist eine qualitative und eine quantitative Auswertung möglich.

### Schnelltest

Das erfindungsgemäße Verfahren zur Bestimmung der Chilischärfe wird alternativ mit einem Schnelltest auf einem Teststreifen durchgeführt. Die Durchführung des Schnelltestes ist für jedermann ohne spezielle Kenntnisse und ohne eine aufwendige Laborausstattung an jedem beliebigen Ort möglich. Das Ergebnis, ob und wieviel Capsaicinoid in einer zu testenden Probe vorliegt, ist einfach und schnell auswertbar. Bevorzugt ist der Schnelltest ein Lateral Flowtest, umfassend eine Dünnschichtchromatografie und eine Immunfärbung.

Mit diesem Schnelltest wird erstmals die zu detektierende Capsaicinoid-Menge qualitativ oder semi-quantitativ erfasst. Die Auswertung beinhaltet eine optische Darstellung z.B. in einer Farbreaktion in den Feldern, die unmittelbar für das menschliche Auge sichtbar ist und direkt als Ergebnis eine Aussage über das Vorhandensein von Capsaicinoid bzw. der Menge von Capsaicinoiden macht.

Vorzugsweise weist der erfindungsgemäße Schnelltest eine Grundfläche aus einem geeigneten Material, wie z.B. Polymer auf. Die Grundfläche ist quadratisch oder rund, vorzugsweise rechteckig. Die Grundfläche ist mit einem für die Dünnschichtchromatografie geeigneten Material beschichtet, z. B. Celluloseacetat oder Cellulosenitrat. Der Schnelltest weist mehrere Zonen auf, insbesondere eine Zone P, in der die zu untersuchende unbekannte Probe aufgebracht wird. Die Probe beginnt nach Zugabe eines geeigneten Laufmittels aufgrund von Kapillarkräften mit der Ausbreitung über die Grundfläche. Die Probe wandert mit der Flüssigkeit zu einer Zone C, wo sich Capsaicin konjugiert mit KLH (=keyhole limpet hemocyanin) oder konjugiert mit BSA befindet sowie getrockneter Erstantikörper, z.B. Huhn IgY, getrockneter enzymgekoppelter zweiter Antikörper, z.B. Anti-Huhn HRP-konjugierter Antikörper und getrocknetes Substrat. Die Flüssigkeit bringt die getrockneten Bestandteile Erstantikörper, Zweitantikörper und Substrat in Lösung, so dass es zu einer Immunreaktion, bei der Capsaicin, also sowohl das konjugierte Capsaicin, als auch die evtl. in der zu testenden Probe vorhandenen Capsaicinoide durch Farbreaktion sichtbar gemacht wird. Je mehr Capsaicinoide in der zu testenden Probe enthalten ist, umso schwächer wird das optische Signal. Die Quantifizierung wird durch eine definierte Menge an konjugiertem Capsaicin realisiert. Alternativ sind Standardzonen S vorgesehen, die unterschiedliche Konzentrationen an konjugiertem Capsaicin aufweisen, so dass die Farbentwicklung wie in einer Standardreihe ermittelt werden kann.

Alternativ ist eine Kontrollzone K vorgesehen, in der keine Probe zugegeben wird, um hier das optische Signal ohne Probenzugabe als Vergleich oder Ausgangssignal zu erhalten. Die Auswertung erfolgt visuell mit dem Auge, durch ein optisches Messgerät (LFT Lesegerät) oder durch eine Smartphone App.

## Patentansprüche

1. Verfahren zur Bestimmung der Chilischärfe einer Probe mittels ELISA-Test umfassend folgende Schritte:
i) Bereitstellen einer Mikrotiterplatte, die in mindestens einer Vertiefung mit konjugiertem Capsaicin beschichtet ist
ii) Blockierung der unspezifischen Bindungsstellen durch Zugabe von Blocklösung in die mindestens eine Vertiefung
iii) Zugabe der zu testenden Probe in die mindestens eine Vertiefung der Mikrotiterplatte
iv) Zugabe eines ersten Antikörpers in die mindestens eine Vertiefung, so dass der erste Antikörper konjugiertes Capsaicin in der Beschichtung und freie Capsaicinoide aus der zu testenden Probe bindet
v) Erstes Waschen der mindestens einen Vertiefung, so dass nicht gebundener erster Antikörper entfernt wird
vi) Zugabe eines zweiten enzymgekoppelten Antikörpers in die mindestens eine Vertiefung der Mikrotiterplatte, so dass der zweite Antikörper an den ersten Antikörper bindet
vii) Zweites Waschen der mindestens einen Vertiefung, so dass nicht gebundener zweiter Antikörper entfernt wird
viii) Zugabe von Substrat in die mindestens eine Vertiefung der Mikrotiterplatte, sodass es durch das am zweiten Antikörper gekoppelte Enzym zu einer enzymatischen Reaktion in ein Produkt kommt
ix) Detektion und Quantifizierung des Produktes und Ermittlung der Chilischärfe aus der zu testenden Probe

2. Verfahren zur Bestimmung der Chilischärfe mittels ELISA-Test gemäß Anspruch 1 **dadurch gekennzeichnet, dass** die Beschichtung mit BSA-konjugiertem Capsaicin oder KLH-konjugiertem Capsaicin erfolgt.

3. Verfahren zur Bestimmung der Chilischärfe mittels ELISA-Test gemäß Anspruch 1 oder 2 **dadurch gekennzeichnet, dass** der erste Antikörper ein polyklonaler Antikörper aus Huhn ist, der Capsaicin und Capsaicinoide bindet.

4. Verfahren zur Bestimmung der Chilischärfe mittels ELISA-Test gemäß einem der Ansprüche 1 bis 3 **dadurch gekennzeichnet, dass** der zweite Antikörper mit Biotin markiert ist und eine separate Zugabe eines an Streptavidin gekoppelten Enzyms erfolgt.

5. Verfahren zur Bestimmung der Chilischärfe mittels ELISA-Test gemäß einem der Ansprüche 1 bis 3 **dadurch gekennzeichnet, dass** der zweite Antikörper mit Meerrettichperoxidase gekoppelt ist.

6. Verfahren zur Bestimmung der Chilischärfe mittels ELISA-Test gemäß einem der Ansprüche 1 bis 3 **dadurch gekennzeichnet, dass** zusätzlich zum zweiten Antikörper ein gegen den zweiten Antikörper gerichteter dritter Antikörper verwendet wird.

7. Verfahren zur Bestimmung der Chilischärfe mittels ELISA-Test gemäß Anspruch 6 **dadurch gekennzeichnet, dass** der dritte Antikörper mit einem Enzym gekoppelt ist.

8. Verfahren zur Bestimmung der Chilischärfe mittels ELISA-Test gemäß Anspruch 6 oder 7 **dadurch gekennzeichnet, dass** der dritte Antikörper mit dem Enzym Peroxidase oder alkalische Phosphatase gekoppelt ist.

9. Verfahren zur Bestimmung der Chilischärfe mittels ELISA-Test gemäß einem der vorangegangenen Ansprüche **dadurch gekennzeichnet, dass** der enzymgekoppelte Antikörper mit einem Substrat umgesetzt wird, das mit Colorimetrie, Chemilumineszenz oder Fluorimetrie detektierbar ist.

10. Verfahren zur Bestimmung der Chilischärfe mittels ELISA-Test gemäß einem der vorangegangenen Ansprüche **dadurch gekennzeichnet, dass** die Detektion und Quantifizierung des Capsaicins durch Colorimetrie, Chemilumineszenz oder Fluorimetrie erfolgt.

11. Verfahren zur Bestimmung der Chilischärfe mittels ELISA-Test gemäß einem der vorangegangenen Ansprüche **dadurch gekennzeichnet, dass** die zu testende Probe ein Pflanzenextrakt oder ein pflanzenextrakthaltiges Lebensmittel ist.

12. Verfahren zur Bestimmung der Chilischärfe mittels ELISA-Test gemäß einem der vorangegangenen Ansprüche **dadurch gekennzeichnet, dass** die zu testende Probe Paprika- oder Chilischoten der Gattung Capsicum umfasst.

13. Verfahren zur Bestimmung der Chilischärfe mittels ELISA-Test gemäß einem der vorangegangenen Ansprüche **dadurch gekennzeichnet, dass** die zu testende Probe ein Wärmepflaster oder Pfefferspray umfasst.

14. Verfahren zur Bestimmung der Chilischärfe mittels ELISA-Test gemäß einem der vorangegangenen Ansprüche **dadurch gekennzeichnet, dass** dieser ein Lateral-Flowtest (LFT) ist.
